# EUROPEAN PATENT APPLICATION

(11) **EP 2 468 188 A2**
(43) Date of publication of application: **27.06.2012**
(21) Application number: 11190399.3
(22) Date of filing: 23.11.2011
(51) Int. Cl.: A61B 6/00

(54) **Portable radiographic image capture device, image capture controller and radiographic image capture system**

(30) Priority: 24.12.2010 JP 2010288644
(71) Applicant: Fujifilm Corporation, Minato-ku Tokyo (JP)
(72) Inventor: Kitano, Kouichi, Kanagawa (JP); Noma, Kentaro, Kanagawa (JP); Ikegame, Yukihisa, Kanagawa (JP)
(74) Representative: Klunker . Schmitt-Nilson . Hirsch

(57) **Abstract**

A portable radiographic image capture device includes: an radiographic image data generating means; a wired communication means that includes a connecting terminal and that performs wired communication with an image capture controller; a wireless communication means; a storing means that stores, as an IP address used to identify the device itself, a first IP address used to perform wired communication and a second IP address used to perform wireless communication; and a setting means that sets the first IP address as the IP address to perform communication with the image capture controller in a case in which the connecting terminal and the image capture controller are wire-connected, and sets the second IP address as the IP address to perform communication with the image capture controller in a case in which the image capture controller is disconnected from the connecting terminal.

## Description

### BACKGROUND OF THE INVENTION

### Field of the Invention

### Description of the Related Art

The present invention relates to a portable radiographic image capture device, an image capture controller, and a radiographic image capture system that can perform wireless communication and wired communication.

### Description of the Related Art

In recent years, a radiation detector such as a flat panel detector (FPD) has been put into practical use in which a radiation-sensitive layer is disposed on a thin film transistor (TFT) active matrix substrate and which thus can directly convert radiation into digital data. A portable radiographic image capture device (hereinafter, also referred to as an "electronic cassette") has been put into practical use which generates by the FPD image information (data) indicating a radiographic image representing applied radiation and which stores the generated image data.

Examples of the electronic cassette include not only an electronic cassette that can perform wired communication by connecting via a cable to an image capture controller (so-called a console), which controls capture of a radiographic image, but also an electronic cassette that can perform wireless communication. Japanese Patent Application Laid-Open (JP-A) No. 2005-6979 discloses a technology of such a electronic cassette that can perform both wired and wireless communication, in which the communication mode is switched by connection and disconnection of a cable.

According to the technology disclosed in JP-A No. 2005-6979, in a case in which a cable is connected to the electronic cassette, a wired communication is initiated, and in a case in which the cable is not connected to the electronic cassette, a wireless communication is initiated. However, in a case in which the electronic cassette has, as an IP address indicating the self device, an IP address used to perform wired communication and an IP address used to perform wireless communication, the technology described in JP-A No. 2005-6979 switches the communication mode, but does not switch the IP address. For this reason, communication with the console cannot be appropriately performed and image capture error may be generated.

### SUMMARY

The invention has been made in view of the above circumferences and provides a portable radiographic image capture device, an image capture controller, and a radiographic image capture system that can automatically set an IP address according to a communication system to the probable radiographic image capture device without an intervention of a user.

A first aspect of the present invention is a portable radiographic image capture device that includes an image data generating means that generates radiographic image data based on irradiated radiation; a wired communication means that includes a connecting terminal and that performs wired communication with an image capture controller that is wire-connected to the connecting terminal; a wireless communication means that performs wireless communication; a storing means that stores, as an IP address used to identify the device itself, a first IP address used to perform wired communication by the wired communication means and a second IP address used to perform wireless communication by the wireless communication means; and a setting means that sets the first IP address as the IP address to perform communication with the image capture controller in a case in which the connecting terminal and the image capture controller are wire-connected, and sets the second IP address as the IP address to perform communication with the image capture controller in a case in which the image capture controller is disconnected from the connecting terminal.

According to the first aspect, if the connecting terminal of the portable radiographic image capture device is wire-connected to the image capture controller, the first IP address which is used to perform the wired communication is set as the IP address used to perform the communication with the image capture controller, and if the image capture controller is disconnected from the connecting terminal, the second IP address which is used to perform the wireless communication is set as the IP address used to perform the communication with the image capture controller. Therefore, the IP address corresponding to the communication mode can be automatically set to the portable radiographic image capture device without an intervention of a user. Thereby, the communication can be prevented from being performed according to an erroneous setting, and it is possible to avoid a situation in which the communication cannot be performed due to the erroneous setting which requires a retake of an image.

A second aspect of the present invention is a radiographic image capture system that includes the portable radiographic image capture device according to the first aspect; and an image capture controller that is configured to be wire-connected to the connecting terminal of the portable radiographic image capture device, that transmits a control signal to the portable radiographic image capture device by wired communication using the first IP address as a transmission destination, and that, if a transmission error has occurred, transmits the control signal by wireless communication using the second IP address as the transmission destination.

Thus, the image capture controller according to the second aspect can transmit the control signal to the portable radiographic image capture device, regardless of which of the first or second IP address is set as the IP address used in the communication with the image capture controller in the portable radiographic image capture device.

A third aspect of the present invention is an image capture controller that includes a connecting terminal that is wire-connected to a portable radiographic image capture device including an image data generating means that generates radiographic image data based on irradiated radiation, a wired communication means that performs wired communication with an image capture controller that is wire-connected thereto, a wireless communication means that performs wireless communication, a storing means that stores, as an IP address used to identify the device itself, a first IP address used to perform wired communication by the wired communication means and a second IP address used to perform wireless communication by the wireless communication means, and a setting means that sets the first IP address or the second IP address as the IP address used to perform communication with the image capture controller, wherein the image capture controller is configured to perform wireless communication and wired communication with the portable radiographic image capture device that is wire-connected to the connecting terminal, and a controlling means that controls the setting means such that the first IP address is set as the IP address of the portable radiographic image capture device in a case in which the connecting terminal and the portable radiographic image capture device are wire-connected, and controls the setting means such that the second IP address is set as the IP address of the portable radiographic image capture device in a case in which the portable radiographic image capture device is disconnected from the connecting terminal.

A fourth aspect of the present invention is an image capture controller including: a connecting terminal that is wire-connected to a portable radiographic image capture device, wherein the image capture controller is configured to perform wireless communication and wired communication with the portable radiographic image capture device that is wire-connected to the connecting terminal, and a controlling means that controls the portable radiographic image capture device such that a first IP address is set as an IP address of the portable radiographic image capture device in a case in which the connecting terminal and the portable radiographic image capture device are wire-connected, and controls the portable radiographic image capture device such that a second IP address is set as the IP address of the portable radiographic image capture device in a case in which the portable radiographic image capture device is disconnected from the connecting terminal.

According to the third and forth aspects, the image capture controller performs control such that the first IP address which is used to perform the wired communication is set as the IP address to the portable radiographic image capture device if the connecting terminal of the image capture controller and the portable radiographic image capture device are wire-connected, and performs control such that the second IP address which is used to perform the wireless communication is set as the IP address to the portable radiographic image capture device if the image capture controller is disconnected from the connecting terminal. Therefore, the IP address according to the communication mode can be automatically set to the portable radiographic image capture device without an intervention of a user. Thereby, the communication can be prevented from being performed according to an erroneous setting, and it is possible to avoid a situation in which the communication cannot be performed due to the erroneous setting which requires a retake of an image.

A fifth aspect of the present invention is a radiographic image capture system that includes a portable radiographic image capture device that includes: an image data generating means that generates radiographic image data based on irradiated radiation; a wired communication means that performs wired communication with an image capture controller that is wire-connected; a wireless communication means that performs wireless communication; a storing means that stores, as an IP address used to identify the device itself, a first IP address used to perform wired communication by the wired communication means and a second IP address used to perform wireless communication by the wireless communication means; and a setting means that sets the first IP address or the second IP address as the IP address to perform communication with the image capture controller; and the image capture controller according to the third aspect or the fourth aspect.

Thus, the image capture controller in the radiographic image capture system according to the fifth aspect operates in the same way as the image capture controller according to the third and forth aspects and controls the settings of the portable radiographic image capture device. Therefore, the IP address corresponding to the communication mode can be automatically set to the portable radiographic image capture device without an intervention of a user. Thereby, the communication can be prevented from being performed according to erroneous setting, and it is possible to avoid a situation in which the communication cannot be performed due to the erroneous setting which requires a retake of an image.

As described above, according to the above aspects, the IP address corresponding to the communication mode can be automatically set to the portable radiographic image capture device without an intervention of a user.

### BRIEF DESCRIPTION OF THE DRAWINGS

Exemplary embodiments of the present invention will be described in detail based on the following figures, wherein:
Fig. 1 is a block diagram illustrating the configuration of a radiographic image capture system according to first and second exemplary embodiments;
Fig. 2A is a diagram illustrating a state in which a tip end of a cable is connected to a connecting terminal of an electronic cassette;
Fig. 2B is a diagram illustrating a state in which the tip end of the cable is connected to the connecting terminal of the electronic cassette;
Fig. 3 is a block diagram illustrating the detailed configuration of the radiographic image capture system according to the first exemplary embodiment;
Fig. 4 is a diagram illustrating setting (configuration) contents of communication information (data) of a communication base station and the electronic cassette in an X-ray room;
Fig. 5 is a flowchart illustrating a process of an address setting program that is executed by a CPU of the electronic cassette;
Fig. 6 is a flowchart illustrating a process of a communication control program that is executed by a CPU of a console;
Fig. 7 is a block diagram illustrating the detailed configuration of a radiographic image capture system according to a second embodiment;
Figs. 8A and 8B are flowcharts illustrating a process of a setting control program that is executed by a CPU of a console in the second embodiment; and
Fig. 8C is a flowchart illustrating a process of an address setting program that is executed by a CPU of an electronic cassette in the second embodiment.

### DETAILED DESCRIPTION

### (First Embodiment)

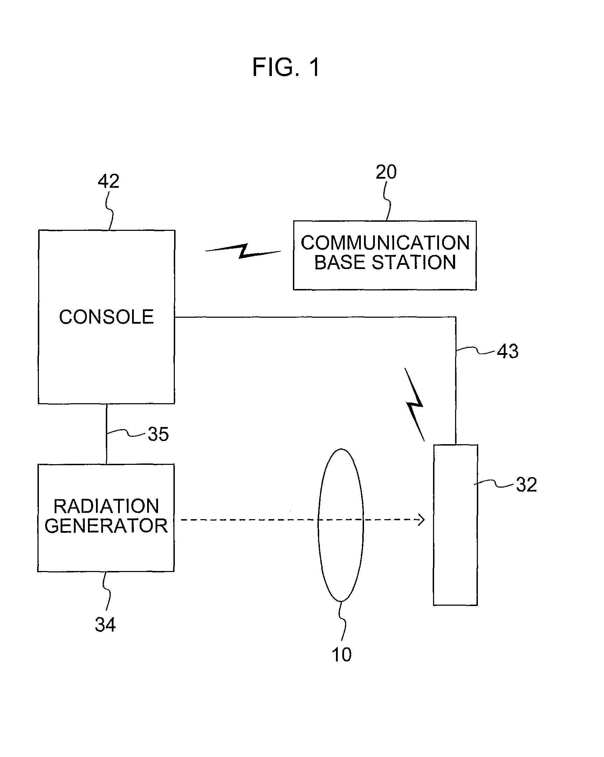

Fig. 1 illustrates an example of the schematic configuration of a radiographic image capture system according to a first embodiment. The radiographic image capture system that is provided in an image capture room (X-ray room) for capturing a radiographic image (in the first embodiment, using X-rays) includes an image capture controller (hereinafter, also referred to as "console") 42, a radiation generator 34, and a radiographic image capture device (hereinafter, referred to as "electronic cassette" or "cassette") 32. A communication base station 20 for performing wireless communication is provided in the X-ray room.

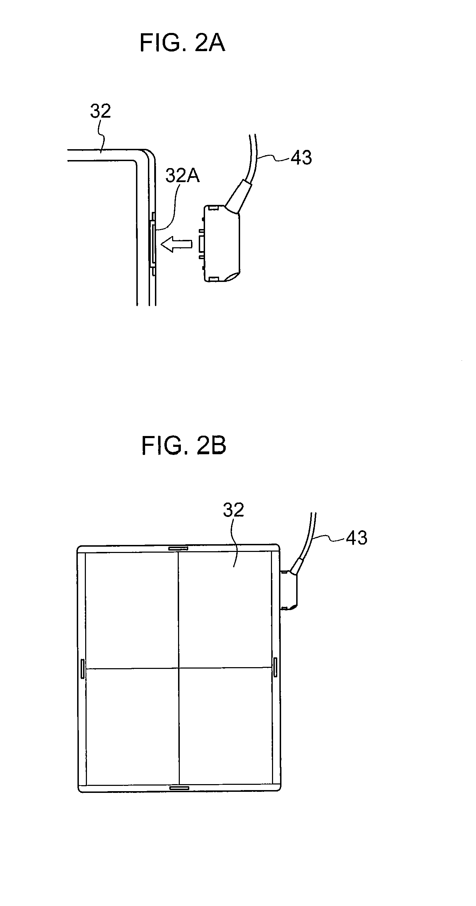

The console 42 is configured to perform communication with the electronic cassette 32 through a communication base station 20. The console 42 and the electronic cassette 32 can perform wired communication when wire-connected via a cable 43. As illustrated in Fig. 2, a connecting terminal 32A is provided in the electronic cassette 32. The console 42 and the electronic cassette 32 are wire-connected when one end of the cable 43 is connected to the console 42, and the other end of the cable 43 is connected to the connecting terminal 32A.

The console 42 performs wired or wireless communication, and transmits a control signal in order to perform various control operations with respect to the electronic cassette 32. The console 42 is connected to the radiation generator 34 through a cable 35 and controls the timing when the radiation is generated.

At a timing based on the control of the console 42, the radiation generator 34 applies radiation onto a subject 10. The radiation that is irradiated from the radiation generator 34 transmits through the subject 10 and is irradiated onto the electronic cassette 32. The electronic cassette 32 generates radiographic image information (data) based on the irradiated radiation. The generated image data is transmitted to the console 42 using wired or wireless communication.

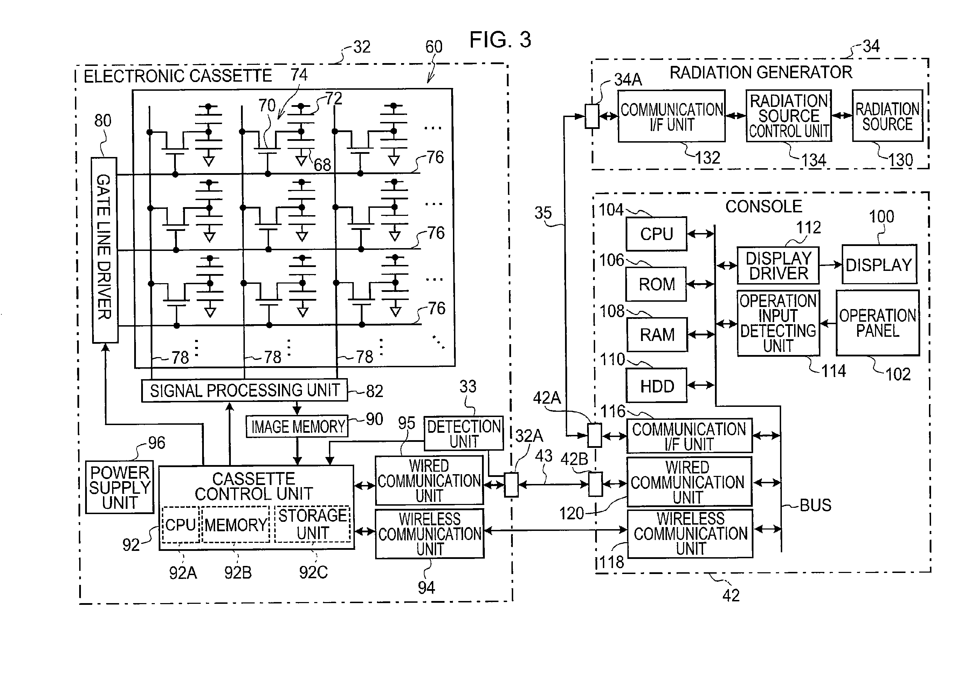

Fig. 3 is a block diagram illustrating the detailed configuration of the radiographic image capture system according to the first embodiment.

The radiation generator 34 includes a connecting terminal 34A for performing communication with the console 42. The console 42 includes a connecting terminal 42A for performing communication with the radiation generator 34 and a connecting terminal 42B for performing communication with the electronic cassette 32.

The radiation generator 34 is connected to the console 42 through the cable 35. The cable 43 is connected to the connecting terminal 32A of the electronic cassette 32 when a radiographic image is captured and the electronic cassette 32 is connected to the console 42 through the cable 43.

A radiation detector 60 that is incorporated in the electronic cassette 32 is configured with a photoelectric conversion layer that is stacked on a TFT active matrix substrate 66, and is for absorbing radiation X and converting the radiation into charges. The photoelectric conversion layer is made of amorphous a-Se (amorphous selenium) containing selenium as a principal component (for example, having content rate of 50% or more). When radiation X is irradiated, the photoelectric conversion layer internally generates charges (pairs of electron and hole) of a charge amount according to the irradiated radiation dose and converts the irradiated radiation X into the charges. Alternatively, the radiation detector 60 may indirectly convert the irradiated radiation X into charges using a phosphor material and a photoelectric conversion element (photodiode), instead of a radiation-charge conversion material such as the amorphous selenium that directly converts radiation X into charges. As the phosphor material, gadolinium oxysulfide (GOS) or cesium iodide (CsI) can be used. In this case, radiation X is converted into light by the phosphor material and the light is converted into charges by the photodiode of the photoelectric conversion element.

On the TFT active matrix substrate 66, plural pixels 74 (in Fig. 3, the photoelectric conversion layer corresponding to each pixel 74 is schematically illustrated as a photoelectric conversion unit 72) each of which includes a storage capacitor 68 that accumulates charges generated by the photoelectric conversion layer and a TFT 70 that reads the charges accumulated in the storage capacitor 68 are arranged in a matrix. The charges that are generated in the photoelectric conversion layer due to irradiation of radiation X onto the electronic cassette 32 are accumulated in the storage capacitor 68 of each of the pixels 74. Thereby, image information that has been carried in the radiation X irradiated onto the electronic cassette 32 is converted into charge information and is held in the radiation detector 60.

The TFT active matrix substrate 66 includes plural gate lines 76 that extend in one direction (row direction) and are used to turn on/off the TFT 70 of each pixel 74, and plural data lines 78 that extend in a direction (column direction) substantially orthogonal to the gate lines 76 and are used to read the accumulated charges from the storage capacitor 68 through a TFT 70 which has been turned on. Each gate line 76 is connected to a gate line driver 80 and each data line 78 is connected to a signal processor 82. When charges are accumulated in the storage capacitor 68 of each pixel 74, the TFT 70 of each pixel 74 is sequentially turned on in a row unit by a signal supplied from the gate line driver 80 through the gate line 76. The charges accumulated in the storage capacitor 68 of the pixel 74 where the TFT 70 is turned on are transmitted as an analog electric signal through the data line 78 and are input to the signal processor 82. Accordingly, the charges accumulated in the storage capacitor 68 of each pixel 74 are sequentially read in a row unit.

Although not illustrated in the drawings, the signal processor 82 includes an amplifier and a sampling/holding circuit that are provided for each data line 78. The charge signal that is transmitted through each data line 78 is amplified by the amplifier and is held in the sampling/holding circuit. A multiplexer and an analog/digital (A/D) converter are sequentially connected to the output end of the sampling/holding circuit, and the charge signal that is held in each sampling/holding circuit is sequentially (serially) input to the multiplexer and is converted into digital image data by the A/D converter.

An image memory 90 is connected to the signal processor 82, and the image data that is output from the A/D converter of the signal processor 82 is sequentially stored in the image memory 90. The image memory 90 has a storage capacity that can store image data indicating a predetermined number of frames' worth of radiographic images, and each time the charges are read line by line, image data corresponding to each read line is sequentially stored in the image memory 90.

The image memory 90 is connected to a cassette controller 92 that controls the entire operation of the electronic cassette 32. The cassette controller 92 is realized by a microcomputer and includes a CPU 92A, memory 92B including ROM and RAM, and a non-volatile storage unit 92C including an HDD or flash memory.

A wireless communication unit 94 and a wired communication unit 95 are connected to the cassette controller 92. The wireless communication unit 94 complies with a wireless local area network (LAN) standard that typically includes the Institute of Electrical and Electronics Engineers (IEEE) 802.11 a/b/g, and controls transmission of various data between external devices and the wireless communication unit 94 using wireless communication. The wired communication unit 95 complies with a wired LAN standard, is connected to the connecting terminal 32A, and controls transmission of various data between the console 42 and the wired communication unit through the connecting terminal 32A and the cable 43. The cassette controller 92 performs transmission and reception of various data between the console 42 and the cassette controller 92 through the wireless communication unit 94 or the wired communication unit 95.

The cassette controller 92 is connected to a detection unit 33. The detection unit 33 is connected to the connecting terminal 32A and detects whether the console 42 is connected to the connecting terminal 32A through the cable 43.

In a case in which the connecting terminal 32A is a connector configured with plural pins, any of the plural pins can be used as a pin used for connection detection and a connection state can be detected by measuring a voltage of the corresponding pin. For example, in a case in which each of the connecting terminal 42A of the console 42 and the connecting terminal 32A of the electronic cassette 32 is a connector configured with ten pins, the first and tenth pins among the ten pins can be used as pins for connection detection. The first and tenth pins of the connecting terminal 42A of the console 42 are short-circuited, the first pin of the connecting terminal 32A of the electronic cassette 32 is grounded (set to 0 V), and the tenth pin of the connecting terminal 32A is pulled up to a predetermined voltage.

In this state, when one end of the cable 43 is connected to the connecting terminal 42A of the console 42 and the other end thereof is connected to the connecting terminal 32A of the electronic cassette 32 and thereby the console 42 is wire-connected to the connecting terminal 32A of the electronic cassette 32e, the voltage of the tenth pin of the connecting terminal 32A is detected as 0 V by the detection unit 33 (because the pins are short-circuited in the console 42).

Further, when the console 42 is disconnected from the connecting terminal 32A (including three cases of: when one end of the cable 43 is disconnected from the connecting terminal 42A of the console 42, when the other end of the cable 43 is disconnected from the connecting terminal 32A of the electronic cassette 32, and when both ends of the cable 43 are disconnected from the connecting terminals 32A and 42A), because the tenth pin of the connecting terminal 32A is pulled up, the pulled-up voltage is detected by the detection unit 33.

In this way, the detection unit 33 can detect the voltage of the pin for connection detection in the connecting terminal 32A and output a detection signal indicating the detected voltage to the cassette controller 92. The cassette controller 92 can recognize whether or not the console 42 is wire-connected to the connecting terminal 32A, using the detection signal.

The method of detecting the wired connection is not limited to the above method. For example, in the LAN specification specifies a signal that is called a link signal, and when a connection is established, the link signal becomes active. Therefore, whether or not the console 42 is connected to the connecting terminal 32A can be detected also by detecting a state of the link signal by a driver of the LAN. Further alternatively, for example, the connecting terminal 32A may be provided with a switch that is turned on when the tip end of the cable 43 is fitted into the connecting terminal 32A and is turned off when the cable 43 is disconnected from the connecting terminal 32A, and a connecting state may be easily detected by detecting a state of the switch. In the case in which this detection method is adopted, one end of the cable 43 always needs to be connected to the connecting terminal 42A of the console 42.

A power supply unit 96 is provided in the electronic cassette 32 and various circuits or elements (the detection unit 33, the gate line driver 80, the signal processor 82, the image memory 90, the wireless communication unit 94, the wired communication unit 95, and the microcomputer functioning as the cassette controller 92) described above operates by power supplied from the power supply unit 96. The power supply unit 96 is charged by power that is supplied through the cable 43 when the cable 43 is connected to the connecting terminal 32A. The power supply unit 96 incorporates a battery (chargeable secondary battery) so that the portability of the electronic cassette 32 is not deteriorated, and supplies power from the charged battery to the various circuits and elements. Although a secondary battery is used as the battery in the present embodiment, embodiments are not limited thereto and the battery may be a primary battery. In Fig. 3, wirings that connect the power supply unit 96 and the various circuits and elements are not illustrated.

The console 42 is configured as a server computer and includes a display 100 that displays an operation menu or a captured radiographic image, and an operation panel 102 that includes plural keys and receives various data or operation instructions.

The console 42 according to the first embodiment includes a CPU 104 that controls an entire operation of the device, a ROM 106 in which various programs including a control program are stored in advance, a RAM 108 that temporarily stores various data, an HDD 110 that stores and maintains various data, a display driver 112 that controls display of various data with respect to the display 100, an operation input detector 114 that detects an operation state with respect to the operation panel 102, a communication interface (I/F) unit 116 that is connected to the connecting terminal 42A and transmits and receives various data such as exposure conditions to be described below between the radiation generator 34 and the communication I/F unit through the connecting terminal 42A and the cable 35, a wireless communication unit 118 that transmits and receives various data between the electronic cassette 32 and the wireless communication unit by the wireless communication, and a wired communication unit 120 that is connected to the connecting terminal 42B and transmits and receives various data between the electronic cassette 32 and the wired communication unit through the connecting terminal 42B and the cable 43.

The CPU 104, the ROM 106, the RAM 108, the HDD 110, the display driver 112, the operation input detector 114, the communication I/F unit 116, the wireless communication unit 118, and the wired communication unit 120 are connected to each other through a system bus BUS. Therefore, the CPU 104 can access to the ROM 106, the RAM 108, and the HDD 110, and can perform control of displaying various data with respect to the display 100 by the display driver 112, control of transmission/reception of various data with the radiation generator 34 by the communication I/F unit 116, control of transmission/reception of various data with the electronic cassette 32 by the wireless communication unit 118, and control of transmission/reception of various data with the electronic cassette 32 by the wired communication unit 120. Further, the CPU 104 can grasp an operation state of a user with respect to the operation panel 102 using the operation input detector 114.

The radiation generator 34 includes a radiation source 130 that outputs radiation X, a communication I/F unit 132 that transmits and receives various data such as exposure conditions between the console 42 and the communication I/F unit, and a radiation source controller 134 that controls the radiation source 130 on the basis of the received exposure conditions.

The radiation source controller 134 is realized by a microcomputer, and stores the received exposure conditions. The exposure conditions received from the console 42 includes data of a tube voltage, a tube current, an irradiation period and the like. The radiation source controller 134 irradiates the radiation X from the radiation source 130 on the basis of the received exposure conditions.

Next, an operation of the radiographic image capture system according to the first embodiment will be described.

The communication base station 20 that is an access point used in the X-ray room holds extended service set identifier (ESSID) and a channel Ch as wireless communication configuration data used to perform wireless communication. The console 42 used in the X-ray room holds the same ESSID and channel Ch as those set in the communication base station 20 of the X-ray room where the console 42 is disposed. The ESSID and the channel Ch are set in advance by being stored in the HDD 110 or the ROM 106 of the console 42. Further, the same ESSID and channel Ch are also set in the electronic cassette 32 that is used in the same radiographic image capture system, by being stored in advance in the storage unit 92C of the electronic cassette 32.

In this case, only one ESSID and only one channel Ch are set to the console 42 and the electronic cassette 32, and plural ESSIDs will never be set to each of the devices.

The communication base station 20 to which the electronic cassette 32 and the console 42 are connected when wireless communication is performed is respectively identified by the ESSID. That is, only the devices which have been set the same set ESSID can perform communication with each other. The wireless communication unit 94 of the electronic cassette 32 performs wireless communication after confirming and authenticating that the ESSIDs of the communication base station 20 and the electronic cassette 32 matches. The wireless communication unit 118 of the console 42 also performs similar conformation and authentication before performing communication.

Further, a frequency band to be used in wireless communication is divided such that plural devices can simultaneously perform communications. The channel Ch indicates these divided frequency bands. In the first embodiment, different channels for devices in different X-ray rooms are assigned to in order to prevent interference of the electric waves between the communication base stations 20. Therefore, the wireless communication unit 94 of the electronic cassette 32 and the wireless communication unit 118 of the console 42 perform wireless communication at the frequency band according to the set channel Ch.

Each of the electronic cassette 32 and the console 42 holds in advance a unique address (in the first embodiment, an IP address, specifically, a local IP address) to identify itself. Specifically, the unique address may be held in the storage unit 92C in the electronic cassette 32, and may be held in the HDD 110 in the console 42. In the first embodiment, the held IP address includes a local IP address for wired communication and a local IP address for wireless communication. Hereinafter, the local IP address for the wired communication is called a wired IP address, and the local IP address for the wireless communication is called a wireless IP address. An IP address that is used as the local IP address used to perform communication needs to be set according to whether the communication mode is wired communication or wireless communication. This setting is stored in a storage area (in the case of the electronic cassette 32, a storage area of the storage unit 92C or a storage area of the memory 92B) that is different from a storage area where the wired IP address and the wireless IP address are stored, and is read to be used at the time of performing communication. In the HDD 110 of the console 42, data of both the wired IP address and the wireless IP address of the electronic cassette 32 which are used in the X-ray room are stored in advance.

In the first embodiment, in a state in which the electronic cassette 32 and the console 42 are wire-connected, a signal transmission and reception for performing an image capture are generally performed by wired communication since the data transmission is stable, and in a state in which the electronic cassette 32 and the console 42 are not wire-connected, the signal transmission and reception for the image capture are performed by wireless communication.

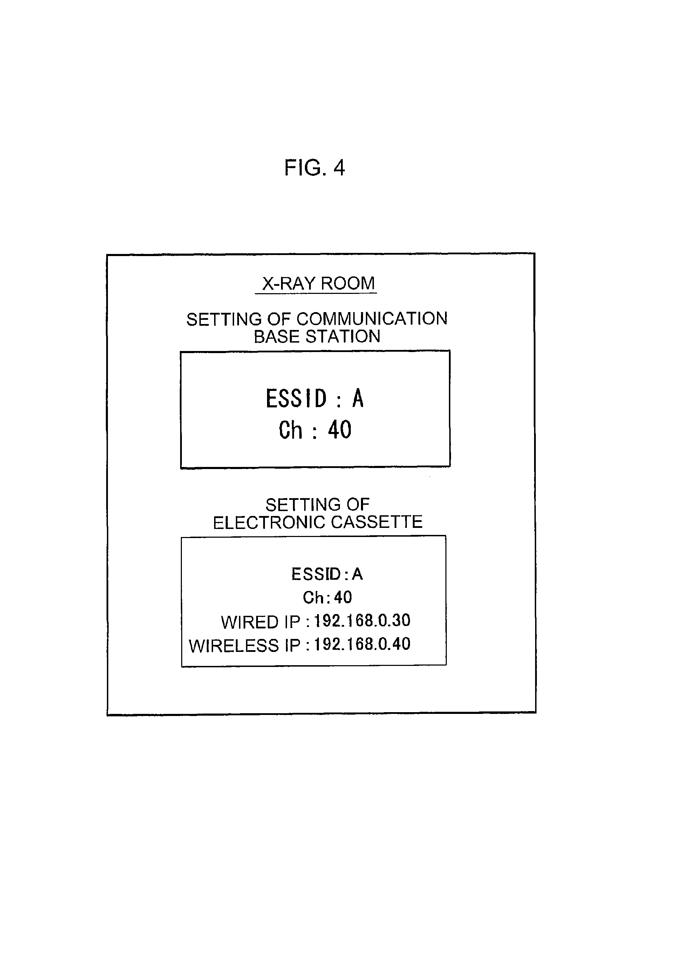

Fig. 4 illustrates an example of the contents of the setting (configuration) of communication data. ESSID "A" and a channel Ch "40" are set to the communication base station 20 that serves as an access point used in the X-ray room as wireless communication setting data which is necessary to perform wireless communication. ESSID "A" and channel Ch "40" which are same as the ESSID and the channel Ch of the communication base station 20 are set to the electronic cassette 32 that is used in the X-ray room. Further, a wired IP address and a wireless IP address that are different from each other are given to the electronic cassette 32.

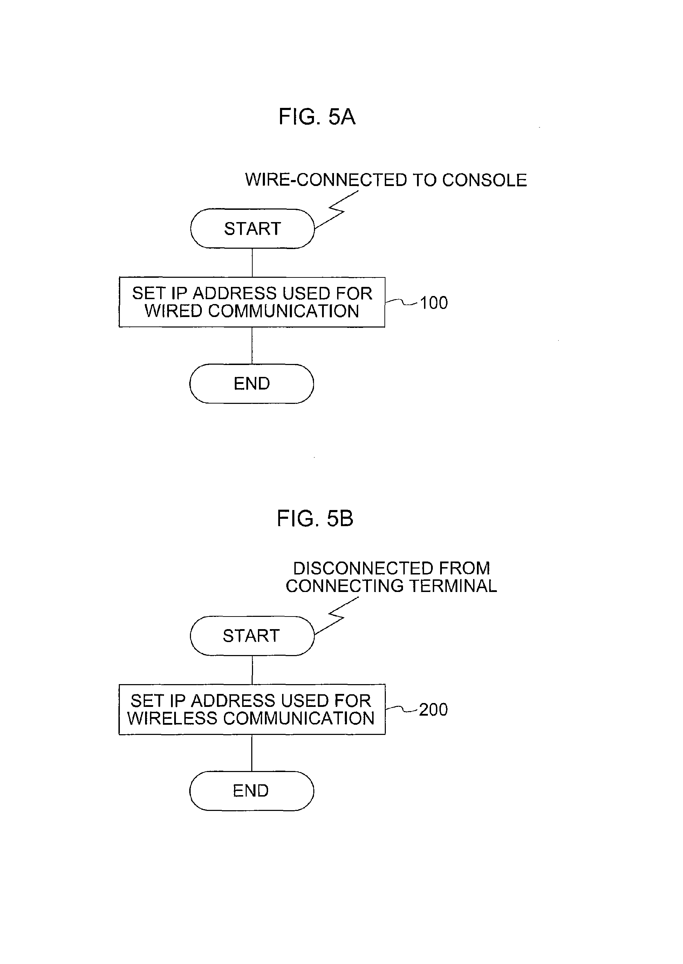

Figs. 5A and 5B are flowcharts illustrating processing of an address setting program that is executed by the CPU 92A of the electronic cassette 32. The address setting program is stored in advance in a predetermined area of the memory 92B (ROM).

If the detection unit 33 detects that the console 42 and the connecting terminal 32A are wire-connected, the CPU 92A performs the program that is illustrated in Fig. 5A. In step 100, an IP address (wired IP address) that is used to perform the wired communication and is held in advance by itself is read and is set as the (local) IP address used to perform the communication with the console 42. In the example illustrated in Fig. 4, a wired IP address 192.168.0.30 is set as the IP address used to perform the communication with the console 42.

However, if the detection unit 33 detects that the console 42 is disconnected from the connecting terminal 32A, the CPU 92A performs the program that is illustrated in Fig. 5B. In step 200, an IP address (wireless IP address) that is used to perform the wireless communication and is held in advance by itself is read and is set as the (local) IP address used to perform the communication with the console 42. In the example illustrated in Fig. 4, a wireless IP address 192.168.0.40 is set as the IP address used to perform the communication with the console 42.

When the electronic cassette 32 performs communication with the console 42, if the console 42 is wire-connected to the connecting terminal 32A, the cassette control unit 92 controls the wired communication unit 95 such that the wired IP address set as the IP address used to perform the communication with the console 42 is used in the communication. If the console 42 is disconnected from the connecting terminal 32A, the cassette control unit 92 controls the wireless communication unit 94 such that the wireless IP address set as the IP address used to perform the communication with the console 42 is used in the communication.

As described above, the electronic cassette 32 sets to the self device the IP address used in the communication with the console 42, according to the wired connection state with the console 42. Therefore, the IP address corresponding to the communication mode can be set automatically without an intervention of a user. Thereby, the communication can be prevented from being performed according to erroneous setting, and the communication can be prevented from being disabled due to the erroneous setting. In addition, the above-described configuration can avoid a situation in which retries of data communication are repeated, and the battery is consumed without performing successful communication, which requires a retake of an image.

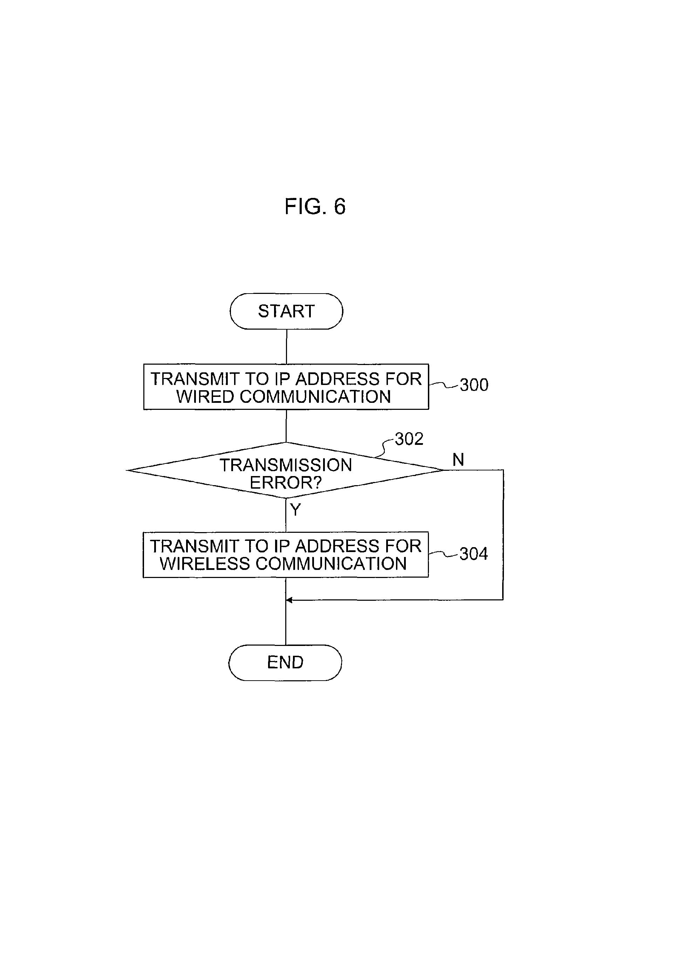

In the first embodiment, the console 42 does not detect whether or not the electronic cassette 32 is wire-connected to the connecting terminal 42A. Therefore, a process illustrated in Fig. 6 may be executed when the console 42 performs communication with the electronic cassette 32. Fig. 6 is a flowchart illustrating a process of a communication control program that is executed by the CPU 104 of the console 42. The communication control program is stored in advance in a predetermined area of the memory 106 (ROM) or the HDD 110.

In step 300, the wired IP address of the electronic cassette 32 is set as a transmission destination and a control signal (for example, control signal for controlling image capture) is transmitted by the wired communication through the wired communication unit 120. If a transmission error occurred at the time of the transmission (YES in step 302), in step 304, the wireless IP address of the electronic cassette 32 is set as the transmission destination and the control signal is transmitted by the wireless communication through the wireless communication unit 118. Thereby, the console 42 can transmit a signal to the electronic cassette 32 regardless of which IP address is set as the local IP address used in the communication with the console 42 in the electronic cassette 32.

### (Second Embodiment)

In the first embodiment, the detection unit 33 connected to the connecting terminal 32A in the electronic cassette 32 detects the wired connection state with the console 42 and the electronic cassette 32 sets the IP address used to perform the communication with the console 42. However, embodiments are not limited thereto. For example, the console 42 may detect the wired connection state with the electronic cassette 32 and a control signal for setting the IP address may be transmitted from the console 42 to the electronic cassette 32.

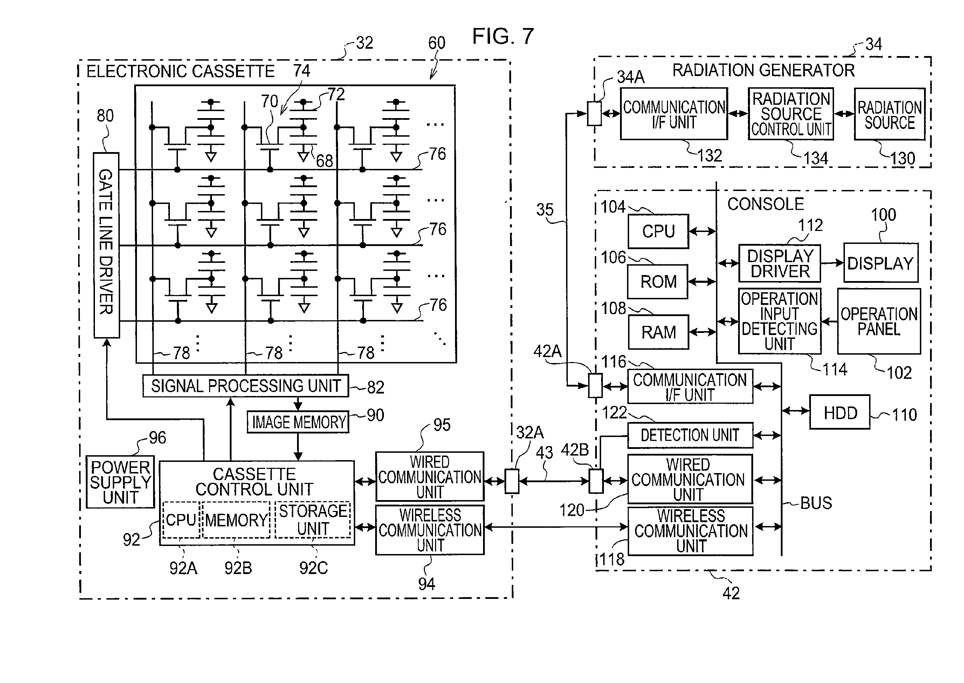

Fig. 7 is a block diagram illustrating the detailed configuration of a radiographic image capture system according to the second embodiment. In Fig. 7, since components denoted by the same reference numerals as those in Fig. 3 have the same functions as those of Fig. 3, duplicated descriptions thereof are omitted.

As illustrated in Fig. 7, in the second embodiment, instead of providing the detection unit 33 in the electronic cassette 32, a detection unit 122 is provided in the console 42. The detection unit 122 of the console 42 is connected to the connecting terminal 42A and detects whether or not the electronic cassette 32 is connected to the connecting terminal 42A through the cable 43 (that is, the detection unit 122 detects the wired connection state with the electronic cassette 32). The result of the detection is transmitted to the CPU 104 through the system bus BUS. Since the detection unit 122 perform the same detecting method as that of the detection unit 33 of the electronic cassette 32 described in the first embodiment, duplicated description thereof will be omitted.

Next, the operation of the radiographic image capture system according to the secondary embodiment will be described.

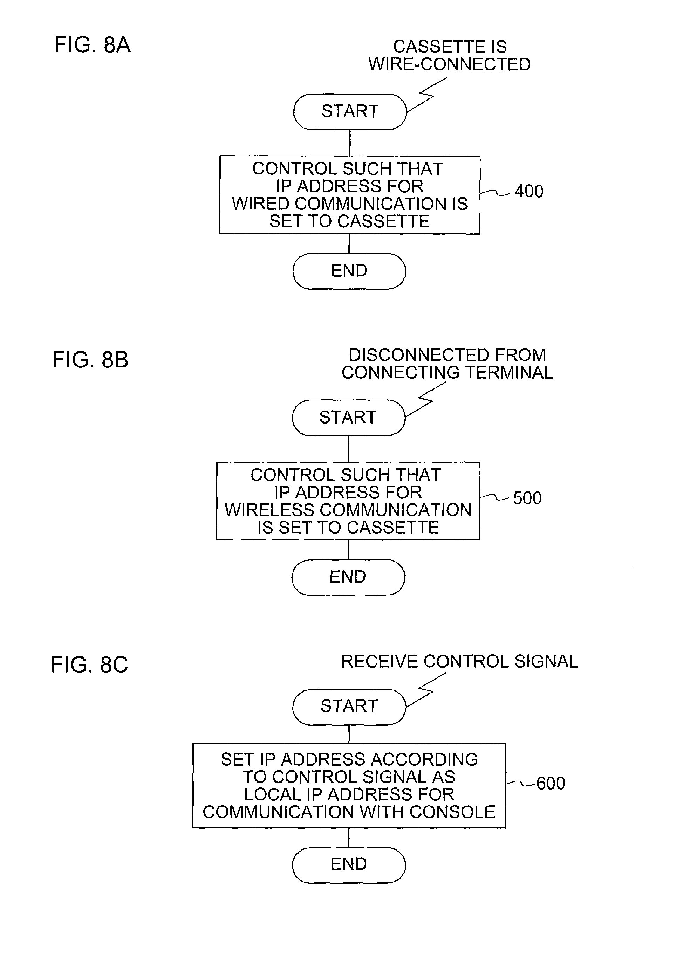

Figs. 8A and 8B are flowcharts illustrating processing of the setting control program that is executed by the CPU 104 of the console 42. The setting control program is stored in advance in a predetermined area of the memory 106 (ROM) or the HDD 110. Fig. 8C is a flowchart illustrating processing of an address setting program that is executed by the CPU 92A of the electronic cassette 32. The address setting program is stored in advance in a predetermined area of the memory 92B (ROM).

If the detection unit 122 of the console 42 detects that the electronic cassette 32 and the connecting terminal 42A are wire-connected, the processing illustrated in Fig. 8A is executed. In step 400, the console 42 transmits a control signal such that the IP address (that is, wired IP address) that is used to perform the wired communication and is held in the electronic cassette 32 is set as the local IP address of the electronic cassette 32 that is used to perform the communication with the console 42.

If the electronic cassette 32 receives the control signal from the console 42, the electronic cassette 32 executes the processing illustrated in Fig. 8C. In step 600, according to the received control signal, the electronic cassette 32 reads an IP address (in this case, wired IP address) that is stored in itself device and sets it as the local IP address that is used to perform the communication with the console 42. In this case, the electronic cassette 32 reads the wired IP address held in itself and sets the wired IP address, according to the control signal. However, embodiments are not limited thereto: the control signal from the console 42 may include data of the wired IP address of the electronic cassette 32, and the electronic cassette 32 may extract the wired IP address from the received control signal and set the wired IP address to itself.

If the detection unit 122 detects that the electronic cassette 32 is disconnected from the connecting terminal 42A, the console performs the processing illustrated in Fig. 8B. In step 500, the console 42 transmits a control signal such that an IP address (that is, wireless IP address) used to perform the wireless communication and held in the electronic cassette 32 is set as the local IP that is address used to perform the communication with the console 42.

If the electronic cassette 32 receives the control signal from the console 42, as same as described above, the electronic cassette 32 executes the processing illustrated in Fig. 8C. In step 600, according to the received control signal, the electronic cassette 32 reads the IP address (in this case, wireless IP address) that is stored in itself device and sets it as the local IP address that is used to perform the communication with the console 42. Also in this case, the control signal from the console 42 may include data of the wireless IP address of the electronic cassette 32, and the electronic cassette 32 may extract the wireless IP address from the received control signal and set the wired IP address to itself.

Alternatively, in Fig. 8A, when the console 42 transmits the control signal, the transmission destination may be set to the wireless IP address of the electronic cassette 32 and the control signal may be transmitted using wireless communication. Before the setting is changed, the electronic cassette 32 is in a state in which the wireless IP address is set as the local IP address used in the communication with the console 42. Therefore, there is no problem in receiving the control signal using wireless communication and perform setting.

The electronic cassette 32 may be configured such that, even in a state in which the local IP address for communication that is set therein is not the wired IP address used for wired communication with the console 42, if the control signal transmitted from the console 42 by wired communication is a predetermined control signal and the transmission destination thereof is set to any of the IP addresses given to the electronic cassette 32, the electronic cassette 32 recognizes the control signal as a signal transmitted to itself and receives the control signal. If the control signal for setting the IP address of the electronic cassette 32 is included in the predetermined control signal, when the control signal is transmitted from the console 42 in Fig. 8A, the wired IP address of the electronic cassette 32 may be set as the transmission destination and the control signal may be transmitted by wired communication. However, these communications are limitative, and various control operations during the image capture operation cannot be executed in a state in which the local IP address set to perform the communication with the console 42 is different from an IP address to be originally set.

Further, when the control signal is transmitted from the console 42in Fig. 8B, the control signal may be broadcasted by wireless communication. In this case, similarly to the above case, the electronic cassette 32 may be configured such that even in a state in which the local IP address for communication that is set therein is not set the wired IP address used for wireless communication with the console 42, if the control signal transmitted from the console 42 by wireless communication is a predetermined control signal and any of the IP addresses given to the electronic cassette 32 is set as the transmission destination, the electronic cassette 32 recognizes the control signal as a signal transmitted to itself and receives the control signal. If the control signal for setting the IP address of the electronic cassette 32 is included in the predetermined control signal, when the control signal is transmitted from the console 42 in Fig. 8B, the wireless IP address of the electronic cassette 32 may be set as the transmission destination and the control signal may be transmitted by wireless communication.

As described above, the console 42 performs the control operations such that the IP address used to perform communication with the console 42 is set to the electronic cassette 32 according to the wired connection state with the electronic cassette 32. Therefore, the same effect as that described in the first embodiment can be obtained.

In the first and second embodiments, the detection unit 33 or the detection unit 122 continuously detects the wired connection state and outputs the detection results. However, embodiments are not limited thereto. For example, the detection unit 33 or the detection unit 122 may detect the wired connection state each time when a predetermined time period elapses, or detect the wired connection state at a predetermined timing (for example, when an activation of the entire system or when a power supply is turned on in the electronic cassette 32).

Further, the electronic cassette 32 may be configured such that the IP address used to perform the communication with the console 42 may be stored and set in non-volatile memory, and regardless of whether the power supply of the system is turned on/off, if the connection state does not change, the communication may be performed using the setting in the non-volatile memory. Alternatively, regardless of whether the setting of the IP address is stored in the non-volatile memory or the volatile memory, when the system is activated (when the power supply is turned on), the console 42 may perform the detection by the detection unit 122 and the IP address may be set to the electronic cassette 32.

In the first and second embodiments, cases in which the console 42 and the radiation generator 34 are provided as separate devices are described. However, embodiments are not limited thereto. For example, the console 42 and the radiation generator 34 may be configured as a single device.

In the first and second embodiments, cases in which one electronic cassette 32 is provided are described. However, embodiments are not limited thereto, and even in a case in which plural electronic cassettes 32 are provided in the radiographic image capture system, the IP addresses thereof can be set by performing the same control as described above.

In the first and second embodiments, X-ray is applied as radiation. However, embodiments are not limited thereto and gamma rays or the like may be applied as radiation.

The configuration (refer to Figs. 1, 3, and 7) of the radiographic image capture system that is described in the first and second embodiments and the shape (refer to Fig. 2) of the cable are examples and the configuration and shape can be changed depending on the situation of application in a range without departing from the gist of the invention.

In addition, the processes (refer to Figs. 5, 6, and 8) of various programs described in the first and second embodiments are examples and the processes can be changed depending on the situation of application in a range without departing from the gist of the invention.

## Claims

1. A portable radiographic image capture device comprising:
an image data generating means that generates radiographic image data based on irradiated radiation;
a wired communication means that includes a connecting terminal and that performs wired communication with an image capture controller that is wire-connected to the connecting terminal;
a wireless communication means that performs wireless communication;
a storing means that stores, as an IP address used to identify the device itself, a first IP address used to perform wired communication by the wired communication means and a second IP address used to perform wireless communication by the wireless communication means; and
a setting means that sets the first IP address as the IP address to perform communication with the image capture controller in a case in which the connecting terminal and the image capture controller are wire-connected, and sets the second IP address as the IP address to perform communication with the image capture controller in a case in which the image capture controller is disconnected from the connecting terminal.

2. A radiographic image capture system comprising:
the portable radiographic image capture device according to claim 1; and
an image capture controller that is configured to be wire-connected to the connecting terminal of the portable radiographic image capture device, that transmits a control signal to the portable radiographic image capture device by wired communication using the first IP address as a transmission destination, and that, if a transmission error has occurred, transmits the control signal by wireless communication using the second IP address as the transmission destination.

3. An image capture controller comprising:
a connecting terminal that is wire-connected to a portable radiographic image capture device including an image data generating means that generates radiographic image data based on irradiated radiation, a wired communication means that performs wired communication with an image capture controller that is wire-connected thereto, a wireless communication means that performs wireless communication, a storing means that stores, as an IP address used to identify the device itself, a first IP address used to perform wired communication by the wired communication means and a second IP address used to perform wireless communication by the wireless communication means, and a setting means that sets the first IP address or the second IP address as the IP address used to perform communication with the image capture controller, wherein the image capture controller is configured to perform wireless communication and wired communication with the portable radiographic image capture device that is wire-connected to the connecting terminal, and
a controlling means that controls the setting means such that the first IP address is set as the IP address of the portable radiographic image capture device in a case in which the connecting terminal and the portable radiographic image capture device are wire-connected, and controls the setting means such that the second IP address is set as the IP address of the portable radiographic image capture device in a case in which the portable radiographic image capture device is disconnected from the connecting terminal.

4. An image capture controller comprising:
a connecting terminal that is wire-connected to a portable radiographic image capture device, wherein the image capture controller is configured to perform wireless communication and wired communication with the portable radiographic image capture device that is wire-connected to the connecting terminal, and
a controlling means that controls the portable radiographic image capture device such that a first IP address is set as an IP address of the portable radiographic image capture device in a case in which the connecting terminal and the portable radiographic image capture device are wire-connected, and controls the portable radiographic image capture device such that a second IP address is set as the IP address of the portable radiographic image capture device in a case in which the portable radiographic image capture device is disconnected from the connecting terminal.

5. A radiographic image capture system comprising:
a portable radiographic image capture device that includes:
an image data generating means that generates radiographic image data based on irradiated radiation;
a wired communication means that performs wired communication with an image capture controller that is wire-connected;
a wireless communication means that performs wireless communication;
a storing means that stores, as an IP address used to identify the device itself, a first IP address used to perform wired communication by the wired communication means and a second IP address used to perform wireless communication by the wireless communication means; and
a setting means that sets the first IP address or the second IP address as the IP address to perform communication with the image capture controller; and
the image capture controller according to claim 3 or 4.
